# EUROPEAN PATENT SPECIFICATION

(11) **EP 0 966 972 B1**
(45) Date of publication and mention of the grant of the patent: **24.09.2003**
(21) Application number: 99304805.7
(22) Date of filing: 18.06.1999
(51) Int. Cl.: A61K 47/24

(54) **Topical composition containing silicon gum**
Topische Zusammensetzung enthaltend Silicon-gummi
Composition topique contenant de la gomme de silicone

(30) Priority: 18.06.1998 EP 98480043
(43) Date of publication of application: 29.12.1999
(73) Proprietor: Dow Corning France S.A., 06904 Sophia Antipolis (FR)
(72) Inventor: Aguadisch, Louis Michel Jacques, 06560 Valbonne (FR); STALET, Gilles, 6250 Mougins (FR); MALLARD, Claire, 6250 Mougins (FR); CHAVEL, Anne-Laure, 6600 Antibes (FR); CAYLUS, Ghislaine, 6810 Auribeau sur Siagne (FR)
(74) Representative: Williams, Paul Edwin

(56) References cited:
- EP-A- 0 249 193

## Description

This invention is concerned with compositions which are useful for delivering pharmaceutically active agents topically to human or animal bodies. Specifically, the compositions of this invention comprise pharmaceutically active agents, controlled amounts of silicone gums to increase the substantivity of the pharmaceutically active agent, and pharmaceutical excipients.

Many topical compositions for delivering pharmaceutically active agents to human or animal bodies are known in the art. These include, for example, ointments, creams, gels, solutions, lotions, transdermal and local patches and film matrices.

Many of these topical dosage forms utilise silicones in their formulations. For instance, silicone oils can be used in ointments and salves, volatile silicones can be used as excipients in many formulations and silicone based materials can be used as matrices or membranes through which pharmaceutically active agents are able to diffuse into the body at a controlled rate.

US patent 5,582,815 teaches the use of a volatile silicone as a carrier in a drug delivery system. This patent teaches that the silicone enables spraying of the drug composition without leaving residual carrier on the body. This reference does not, however, teach a silicone gum for forming a substantive film.

French patent application 94 05272 also teaches a silicone containing topical dosage form comprising a lipophilic active substance, a silicone based adhesive polymer composition and a volatile solvent. This reference, however, requires the use of a large amount of silicone adhesive polymer. These large amounts are not only expensive, but they also make dissolution of the active in the solvent more difficult; they result in a film with inferior aesthetics; they prevent the formulations from being sprayed; and they require a long time for drying.

We have now discovered a composition useful for delivering a pharmaceutically active agent topically to a human or animal body which avoids the problems of the prior art.

The present invention provides in one of its aspects a composition for delivering a pharmaceutically active agent topically to a human or animal body comprising 0.1 to 25 wt. % pharmaceutically active agent; 0.1 to 3 wt. % silicone gum; and 72 to 99.8 wt. % pharmaceutical excipient.

The compositions of this invention provide a substantive, silky feeling film on the human or animal body from which the pharmaceutically active agent is released. These compositions also inhibit caking of formulations in which the pharmaceutically active agent is insoluble.

The compositions of the present invention contain a pharmaceutically active agent. Generally, the pharmaceutically active agent chosen is not critical. It can comprise any solid or liquid material for which topical delivery to a human or animal body is desired. As used herein, 'topical' means not only active agents intended for the skin but also those intended for ocular, buccal, nasal, aural, vaginal or rectal cavity or a cavity formed, for example, in a tooth or an open wound. These active agents include therapeutic agents or diagnostic agents.

Therapeutic agents which may be employed include, for example, antibiotics, antiseptics, antifungal, antiacne, antiinflammatory agents, hormones, anticancer agents, smoking cessation agents, cardiovasculars, H₂ blockers, bronchodilators, analgesics, antiarrythmics, antihistamines, alpha blockers, beta blockers, ACE inhibitors, diuretics, antiaggregants, sedatives, tranquillisers, anticonvulsants, anticoagulants, vitamins, agents for treating gastric and duodenal ulcers, proteolytic enzymes, healing factors, cell growth nutrients, peptides and others. Specific examples of suitable therapeutic agents include penicillins, cephalosporins, tetracyclines, macrolides, epinephrine, amphetamines, aspirin, barbiturates, catecholamines, benzodiazepine, thiopental, codeine, morphine, procaine, lidocaine, sulphonamides, ticonazole, perbuterol, furosamide, prazosin, prostaglandins, salbutamol, indomethacine, diclofenac, glafenine, dipyridamole, and theophylline. Mixtures of pharmaceutically active agents can also be used herein.

Suitable diagnostic agents are also known in the art and can include, for example, materials opaque to x-rays.

The proportion of the pharmaceutically active agent employed in a composition according to the invention is chosen in accordance with the concentration of the pharmaceutically active agent required to deliver the dosage required. This may vary within a wide range such as from 0.1 to about 25 weight percent of the composition. Generally, however, the pharmaceutically active agent will comprise about 0.1 to about 10 wt. % of the composition.

The silicones gums used in the present invention are known in the art and many are commercially available. Generally, they comprise silicone polymers and have the structure:

R₃Si-O- [R₂Si-O]ₙ-SiR₃

In this structure, each R is independently a hydrogen, a hydroxyl, an alkoxy or a hydrocarbon of 1-20 carbon atoms such as methyl, ethyl, propyl, vinyl, phenyl, etc. Generally preferred are polymers in which the repeating units comprise dimethyl, methylvinyl, methylphenyl, methylhydrogen, methylhydroxyl and mixtures thereof and the end-capping units comprise dimethylhydroxyl, dimethylvinyl, trimethyl and methylvinylphenyl. Most preferred are silicone gums having dimethylsiloxy repeating units terminated with trimethylsilyl or dimethylhydoxylsilyl groups. Such materials and methods for their preparation are described, for example, in Chemistry and Technology of Silicones, W. Noll, Academic Press, New York, 1968.

Silicone gums typically have viscosities up to 50 million mm²/s at 25°C and have number average molecular weights (Mn) of up to 700,000 or more. Preferably, the gums have an Mn of greater than about 200,000, more preferably greater than about 300,000 and most preferably about 200,000 to 500,000.

Generally, the silicone gum is used in a concentration of about 0.1 to about 3 wt. %, preferably 0.5 to 2.85 wt. %. If less than 0.1 wt % of this agent is used, generally the composition has poor substantivity. If more than 3 wt. % of this agent is used, the composition has poor aesthetics, it interferes with pharmaceutical active agent dissolution and spraying, and it delays the drying time for the formulations.

The pharmaceutical excipient used herein is not critical as long as it can deliver the pharmaceutically active agent and the silicone gum to the desired site by the desired means. The excipient can comprise one or more materials. Examples of such excipients include water, solvents, diluents, excipients used to make emulsions, excipients used to make gels, excipients used to make lotions, excipients used to make ointments, excipients used to make creams, and the like. These excipients are commonly used in compounding pharmaceutical products and can be made by known methods.

Specific examples of materials to be used as excipients include water, alcohols, polyols, ethers, esters, aldehydes, ketones, fatty acids and alcohols, fatty esters,
mineral oils, hydrocarbon oils, oleaginous bases, silicone oils and volatiles, waxes, essential oils, mono and poly saccharides, natural gums, cellulose derivatives, acrylic acid polymers, polyvinyl alcohol, polyvinyl pyrrolidone, proteins, polyethylene glycols and copolymers, colloidal solids, surfactants (nonionics, ionics and amphoteric), preservatives, antioxidants, buffering agents, penetration enhancers, propellants, dyes, flavouring agents, cosmetics such as perfumes and coloring agents, terpenes, urea, cyclodextrins, silicone copolyols and the like.

The total amount of pharmaceutical excipient generally used herein is between about 72 and 99.8 wt % of the composition. The pharmaceutical excipient can be one material (e.g., a solvent) or it can be a mixture of materials (e.g., materials used to make a cream). If the excipient is more than one material, the total amount of all the excipients combined is between about 72 and 99.8 wt. %. For instance, if the pharmaceutically active agent and the silicone gum are to be delivered in a cream form, the pharmaceutical excipient would comprise a mixture of those materials commonly used in the manufacture of a cream and the total amount of such materials would be in the range specified above.

Volatile excipients are often preferred herein. Generally, volatile excipients should be sufficiently volatile to evaporate in sufficient time (e.g., < 15 minutes) to leave the substantive film.

Examples of suitable volatile excipients include silicon containing materials such as hexamethyldisiloxane, octamethyltrisiloxane, decamethyltetrasiloxane and other short chain linear siloxanes, cyclic siloxanes such as octamethylcyclotetrasiloxane and decamethylcyclopentasiloxane, organic materials such as alkanes, alcohols, or ketones, mixtures of such materials or any other material which can dilute the formulation without affecting any of the components of the formulation. Preferred herein is hexamethyldisiloxane.

If desired the formulation may contain additional ingredients such as fillers (which may be, for example, opaque to X rays or other diagnostic radiation), colorants, coloured indicators, extenders such as silicone fluids, silicone resins, other excipients employed in pharmacy, compounds intended to perform as pH buffers in controlling the environment immediately in and around the formulation, processing aids such as cyclic or linear polydiorganosiloxanes, bioadhesive materials and the like.

The present invention also relates to a method of forming a substantive film comprising mixing components comprising 0.1 to 25 wt. % pharmaceutically active agent; 0.1 to 3 wt. % silicone gum; and 72 to 99.8 wt. % pharmaceutical excipient to form a composition; and applying the composition to a substrate, preferably a human or animal body, to form the substantive film on the substrate. If a volatile excipient is used, the additional step of allowing the volatile excipient to evaporate is often used in the deposition process.

The compositions according to the invention can be applied in any manner desired. For instance, the composition can be applied by hand, by immersion, by spraying and the like.

If a volatile excipient is used, it is often preferred to spray the composition of the invention. The spraying herein is performed by conventional techniques well known in the art. For instance, the composition can be sprayed by a mechanical sprayer which mechanically pumps the compositions from a container. Similarly, the composition can be forced from a container by a propellant as is well known in the art. Conventional propellants such as air or hydrocarbons will function herein as long as they don't interfere with the formulation. The composition is generally forced through a nozzle which can be directed at the location desired for applying. The nozzle can merely direct a stream of the composition at the desired site or it can create droplets of the formulation by techniques known in the art.

Depending on the pharmaceutically active agent, the spraying mechanism often must be capable of delivering a controlled amount of the composition such that the appropriate dose of the pharmaceutically active agent is delivered. Mechanisms for controlling the amount of compositions delivered by a spray are known in the art. For instance, the mechanical pump can be designed to deliver a controlled quantity of composition with a metering valve. Similarly, the amount of propellant used with an activation can be controlled such that discrete quantities of composition are expelled.

The present invention offers numerous advantages over the prior art. The method described herein allows for the simple dispensing of a pharmaceutical composition which will adhere to a substrate (substantivity) for a sufficient time to release said pharmaceutical agent to said substrate. As such, a skilled practitioner is not required for application. Likewise, small amounts of silicone gum are used so that costs are minimised and aesthetics are improved. Moreover, the amount of silicon gum is small enough that it can be sprayed when a volatile excipient is used.

In order that the invention may become more clear there now follows a description of a composition illustrative of the invention. Unless indicated, all parts are by weight and all viscosities are at 25°C.

### Example 1-3 Placebo

In a container was mixed 5 wt. % talc, silicone gum (average dp = 9500, molecular weight = 700,000, viscosity at 25°C = 0.45 Pa s) in the amounts indicated in Table 1 and hexamethyldisiloxane (HMDS) (molecular weight = 162, viscosity = 0.65 mm²/s at 25°C) in the amount indicated in Table 1.

Delivery of these compositions by spraying using a mechanical sprayer and a conventional mechanical metering valve was attempted. The results are presented in Table 1:

**Table 1**

| Ex. | Wt. % Silicone Gum | wt. % HMDS | Results |
|---|---|---|---|
| 1 | 1 | 94 | Sprayable * |
| 2 | 2 | 93 | Sprayable * |
| 3 | 5 | 90 | Not Sprayable |

| | | | |
|---|---|---|---|
| * The HMDS evaporated in 2 minutes leaving the substantive film on the skin. | | | |

This Example demonstrates that compositions containing low amounts of silicone gum can be sprayed whereas those with higher amounts cannot.

### Examples 4-12

The following compositions were made in the same manner as Example 1. The Silicone Gum and HMDS are the same as in Example 1:

| | | |
|---|---|---|
| 4. (control) | Silicone Gum | 3% |
| | HMDS | 97% |
| | | |
| 5. (control) | Ketoprofen | 8% |
| | Ethyl alcohol | 18.4% |
| | HMDS | 73.6% |
| | | |
| 6. | Ketoprofen | 8% |
| | Ethyl alcohol | 18.4% |
| | Silicone Gum | 0.7% |
| | HMDS | 72.9% |
| | | |
| 7. | Ketoprofen | 8% |
| | Ethyl alcohol | 18.4% |
| | Silicone Gum | 2.2% |
| | HMDS | 71.4% |
| | | |
| 8. (control) | Ketoprofen | 2.5% |
| | Ethyl alcohol | 19.5% |
| | HMDS | 78% |
| | | |
| 9. | Ketoprofen | 2.5% |
| | Ethyl alcohol | 19.5% |
| | Silicone Gum | 0.8% |
| | HMDS | 77.2% |
| | | |
| 10. | Ketoprofen | 2.5% |
| | Ethyl alcohol | 19.5% |
| | Silicone Gum | 1.6% |
| | HMDS | 76.4% |
| | | |
| 11. | Ketoprofen | 2.5% |
| | Ethyl alcohol | 19.5% |
| | Silicone Gum | 2.3% |
| | HMDS | 75.7% |
| | | |
| 12. | Ketoprofen | 2.5% |
| | Ethyl alcohol | 19.5% |
| | Silicone Gum | 2.4% |
| | HMDS | 75.6% |

### Examples 13-15

The following compositions were made in the same manner as Example 1 to show the compatibility of the silicone gum and volatile excipient with other conventional pharmaceutical excipients. The Silicone Gum and HMDS are the same as in Example 1.

| | | |
|---|---|---|
| 13. | Ketoprofen | 1.2% |
| | Menthol | 0.03% |
| | Ethyl alcohol | 7.5% |
| | Silicone Gum | 2.7% |
| | HMDS | 88.5% |
| | | |
| 14. | Ketoprofen | 1.2% |
| | Menthol | 0.03% |
| | Stearyl alcohol | 0.03% |
| | Ethyl alcohol | 7.5% |
| | Silicone Gum | 2.7% |
| | HMDS | 88.5% |
| | | |
| 15. | Ketoprofen | 1.2% |
| | Menthol | 0.03% |
| | Oleic acid | 0.07% |
| | Ethyl alcohol | 7.5% |
| | Silicone Gum | 2.7% |
| | HMDS | 88.5% |

All the formulations were clear.

### Examples 16-17 Solid Formulations

The following solid compositions were made by blending the components listed below in a beaker. The Silicone Gum and HMDS are the same as in Example 1:

| | | |
|---|---|---|
| 16. (control) | Carbopol 980 NF | 0.68% |
| | NaOH 1N | 2.47% |
| | Ketoprofen | 2.45% |
| | Ethyl alcohol | 13.8% |
| | HMDS | 16.3% |
| | Deionized Distilled Water | 64.4% |
| | | |
| 17. | Carbopol 980 NF | 0.68% |
| | NaOH 1N | 2.56% |
| | Ethyl alcohol | 13.8% |
| | Ketoprofen | 2.43% |
| | Silicone Gum | 2.85% |
| | HMDS | 13.3% |
| | Deionized Distilled Water | 64.3% |

### Examples 18-21 Influence of the Silicone Gum Content on the Evaporation of Formulations

The following formulations were made in the same manner as Example 1. The Silicone Gum and HMDS are the same as in Example 1:

| | Control | Example 18 | Example 19 | Example 20 | Example 21 |
|---|---|---|---|---|---|
| Silicone | 0wt% | 10.7wt% | 5.1wt% | 2.94wt% | 1.07wt% |
| Gum HMDS | 100wt% | 89.3wt% | 94.9wt% | 97.06wt% | 98.93wt% |

Each of these formulations were sprayed onto a 47.2 cm² metallic cup at room temperature in a closed balance using a metering dose of 130 µl. The distance from spray nozzle to cup was 5 cm in each case. The cup was weighed prior to spraying, 3 seconds after spraying, and every 30 seconds thereafter until all of the HMDS contained in the formulation had evaporated. The time for evaporating 90 % of the HMDS (T 90) was recorded for each formulation. The control (0 wt.% silicone gum) was used to calculate the % increase in T 90 which occurred as the wt. % silicone gum increased. The results are presented in Table 2.

**Table 2**

| % of Gum | T 90 min | % increase in T90 |
|---|---|---|
| 0 | 5.97 | 0 |
| 1.07 | 6.55 | 9.7 |
| 2.94 | 8.4 | 40.7 |
| 5.1 | 9.08 | 52.1 |
| 10.7 | 9.89 | 65.7 |

These results show that increasing the silicone gum content above 1 wt. % results in a sharp decrease in the rate of evaporation for the HMDS. A gum content of about 3 wt % provides the maximum increase in T90 generally acceptable in the industry. Above this silicone gum content, the evaporation of the volatile excipient is adversely impacted by the silicone gum load and this limits the practical benefit of the topical formulation.

### Example 22 Substantivity of Silicone Gum Formulation on Skin

The forearms of volunteers were cleaned and then sprayed in several areas with the formulation of Example 4. The formulation was allowed to dry for 15 minutes. A FT-IR/ATR cell was used to take an IR spectrum at each area sprayed. An IR spectrum was taken on different areas of the sprayed skin at different times (between 30 minutes and 8 hours as set forth in the table). An IR of the cleansed forearm skin was used as a control blank.

The percentage of silicone remaining on the skin was calculated by comparing the height of the specific silicon peak height (1255 cm⁻¹)/ specific skin peak height (1538 cm⁻¹) in the blank test. The results are shown in Table 3.

**Table 3**

| Time (hr) | AVERAGE (%) | STD DEVIATION |
|---|---|---|
| 0.25 | 100 | 0.0 |
| 0,5* | 62.4 | 17.1 |
| 0,75* | 60.2 | 22.6 |
| 1 * | 65.8 | 21.2 |
| 1.5 | 55.2 | 12.2 |
| 2 | 58.8 | 9.7 |
| 3 | 42.9 | 12.0 |
| 4 | 36.0 | 10.9 |
| 6 | 21.8 | 5.3 |
| 8* | 25.2 | 12.7 |

| | | |
|---|---|---|
| Number of panelist : n = 5 or n = 4 when * | | |

These results clearly show that the substantivity of the silicone gum is decreasing with time. However, after 8 hours about 25% silicone gum still remains. For comparison, HMDS is not detectable on the skin 10 seconds after spraying.

### Example 23 Substantivity of Ketoprofen on Skin with Silicone Gum

The forearm of a volunteer was cleaned and then sprayed in 10 areas with approximately 40 mg of the formulation of Examples 8 (control) and 12. The formulation was allowed to dry for 15 minutes. At selected times (as indicated in the Table 4), 5 pieces of SEBUTAPE (Monaderm Laboratory) were consecutively applied to and stripped from one area of treated skin. A FT-IR/ATR cell was used to take a spectrum of the tapes. An IR of a piece of unused tape was used as a control.

The relative amount of Ketoprofen on the tape was determined by comparing the height of the drug peak to the height of the peak of the unused SEBUTAPE peak on each tape. The relative amounts for the 5 tapes were then added and the results compared vs. time. The results are provided in Table 4.

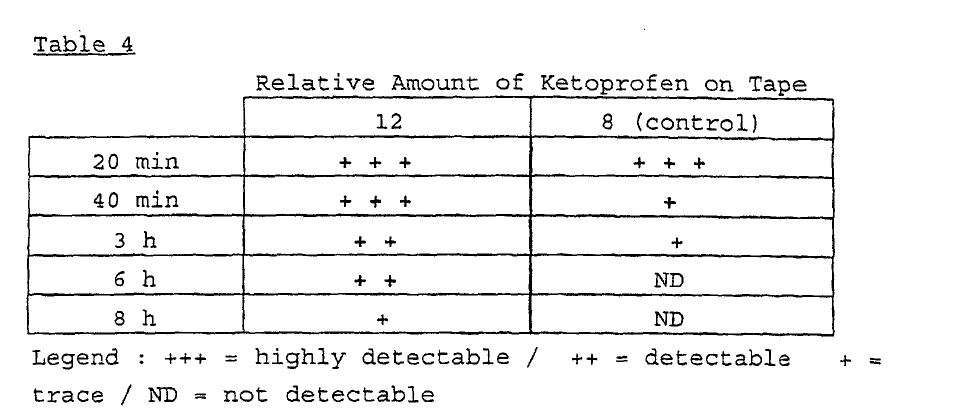

These results show the substantivity of drug on the skin with formulations of the present invention. For the control formulation (8), a significant amount of drug remains on the skin 20 minutes after application and some drug is detectable at 3 hours. After 6 h, however, the drug is not detectable.

By contrast, 40 minutes after application of the formulation of the present invention (12), a significant amount of drug remains on the skin and some drug is still detectable on the skin at 8 hr.

### Example 24 Reservoir Effect of Ketoprofen on Skin with Silicone Gum

The forearm of a volunteer was cleaned and then sprayed with approximately 40 mg of the formulation of Examples 8 (control), 9, 10 and 11. 5 minutes after application, 5 pieces of SEBUTAPE (Monaderm Laboratory) were consecutively applied to and stripped from the treated skin. A FT-IR/ATR cell was used to take an IR spectrum of the tapes. An IR of unused tape was used as a control.

The ratio of the height of the drug peak to the height of the unused SEBUTAPE peak (reference) was determined for each tape. The results are as presented in Table 5.

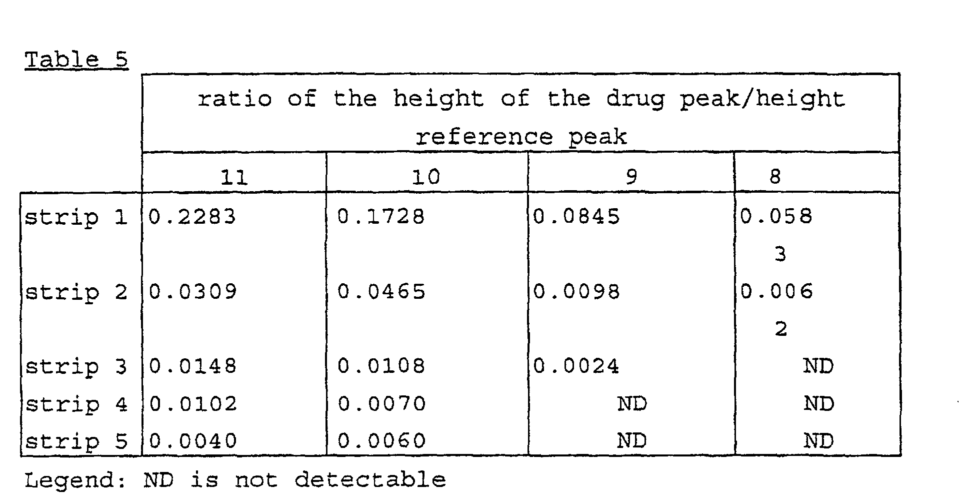

### Example 25 Abrasion Resistance of Ketoprofen on Skin with Silicone Gum

The forearm of a volunteer was cleaned and then sprayed with approximately 40 mg of the formulation of Examples 5 (control), 6 and 7. Approximately 40 mg of the formulation of Examples 5, 6 and 7 was also applied to SEBUTAPE as a 100% reference. Approximately 40 mg of a placebo formulation (no drug) was also applied to SEBUTAPE as a blank test.

5 minutes after application, pieces of SEBUTAPE (Monaderm Laboratory) were consecutively applied to and stripped from the treated skin until no drug remained.

The strips of tape were each transferred to a 50 ml erlenmeyer flask containing a magnetic stirring rod. 10 ml of ethanol was dispensed into the flask and a top applied. The flasks were put into an oven at 40°C and the magnetic stirrer was run at 550 rpm for 1 hour.

The amount of drug in the extract was then calculated using a UV spectrophotometer at 253 nm and compared with the 100% reference. The results are presented in Table 6.

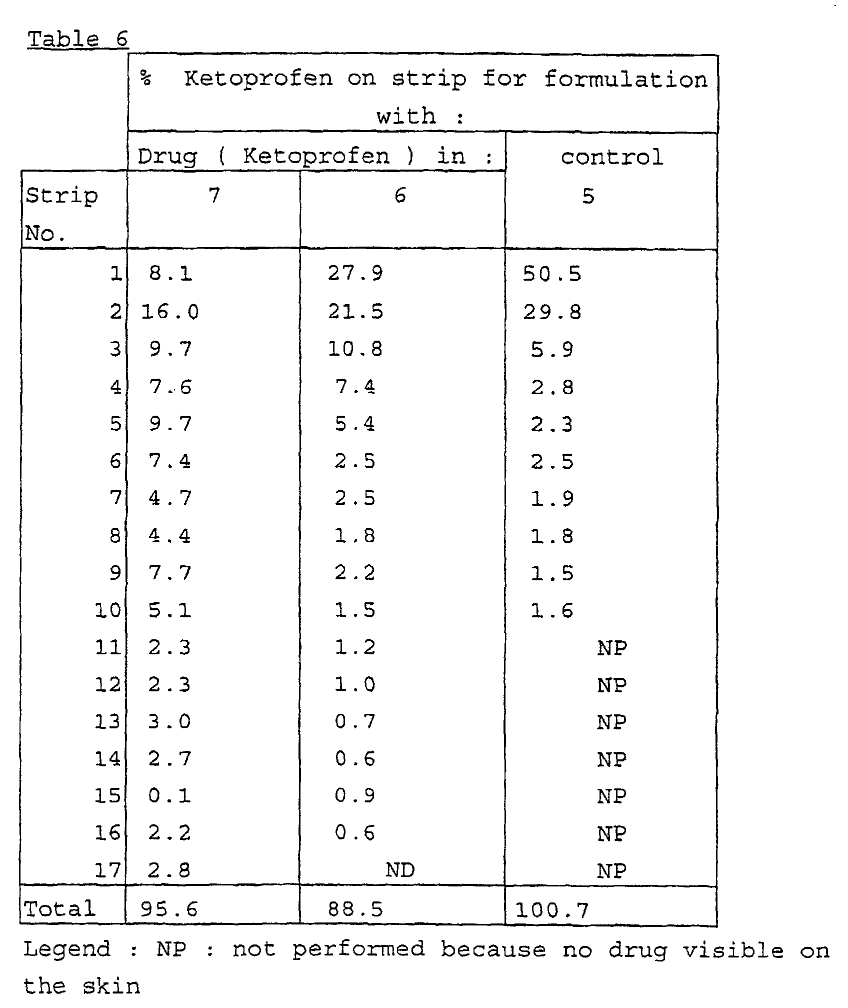

These results show that 10 strips were needed to remove the drug deposited on the skin from the control (5) vs. 17 strips for the formulation containing 2.2% silicone gum (7). About 50 % of the drug is removed by the first strip for the control (5) vs. 28 % for the formulation containing 0.7% silicone gum (6) and 8% for the formulation containing 2.2% silicone gum (7). This clearly indicates the abrasion resistance (substantivity) of the formulations of the invention.

### Example 26 Substantivity of Drug after Application from a Solid Formulation

The forearm of a volunteer was cleaned and then 40 mg of the formulation of Examples 16 (control) and 17 was applied in 2 areas each. At 3 hours and 6 hours after application, 7 pieces of SEBUTAPE (Monaderm Laboratory) were consecutively applied to and stripped from one area of skin treated with the control (16) and one are of the skin treated with the formulation of the invention (17). The drug remaining on the skin after the stripping was collected using a piece of cotton and alcohol. Approximately 40 mg of each formulation was applied on a piece of SEBUTAPE as 100 % control.

Each strip of tape and piece of cotton were transferred to a 50 ml erlenmeyer flask containing a magnetic stirring rod. 10 ml of ethanol was dispensed into the flask and a top applied. The flasks were put into an oven at 40°C and the magnetic stirrer was run at 550 rpm for 1 hour.

The amount of drug in the extract was then calculated using a UV spectrophotometer at 253 nm and compared with the 100% control. The results are present in Table 7.

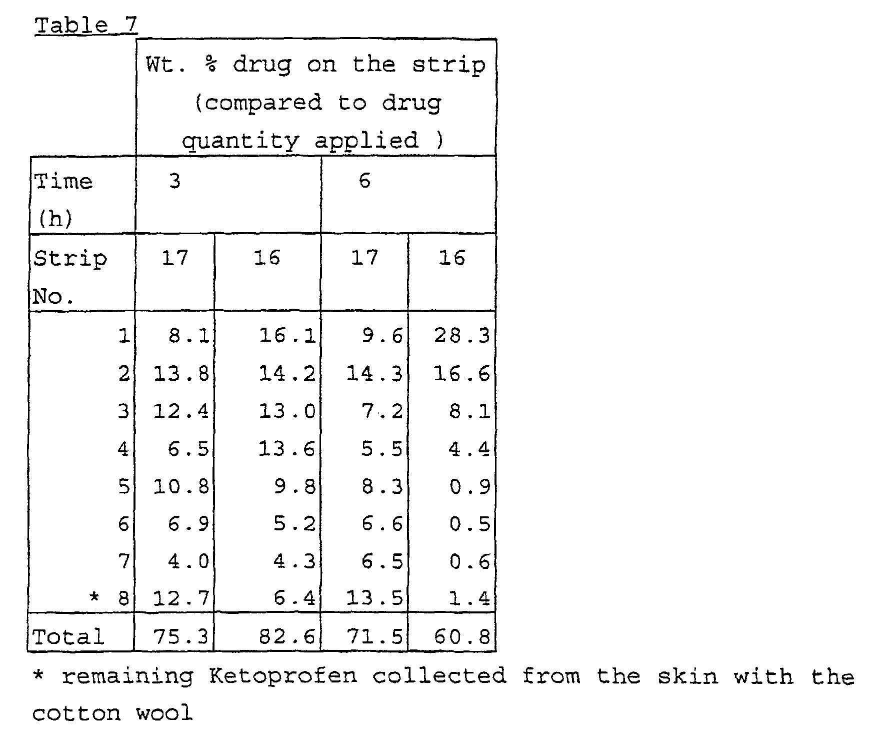

These results indicate that there is no significant difference in substantivity between the formulation of the invention (17) and that of the control (16) 3 hours after application. (Quantity of drug collected on the equivalent strip and total quantity of drug collected for both formulations).

At 6 hours after application, however, the formulation of the invention (17) is more substantive than that for the control formulation (16) as exemplified by the fact that
72 % of the drug applied with the formulation of the invention (17) remains vs. 61 % for the control (16); approximately 51 % of the drug collected is removed with 4 strips for the formulation of the invention (17) vs. 94 % for the control (16); and 13 % of the drug remains on the skin after the 7th strip for the formulation of the invention (17) vs. about 1 % for the control (16).

## Claims

1. A composition for delivering a pharmaceutically active agent topically to a human or animal body comprising:
0.1 to 25 wt. % pharmaceutically active agent;
0.1 to 3 wt. % silicone gum; and
72 to 99.8 wt. % pharmaceutical excipient.

2. A composition according to Claim 1 wherein the pharmaceutically active agent is selected from the group consisting of diagnostic agents, antibiotics, antiseptics, antifungal, antiacne, hormones, anticancer agents, smoking cessation compositions antiinflammatories, cardiovasculars, H₂ blockers, bronchodilators, analgesics, antiarrythmics, alpha-1 blockers, beta blockers, ACE inhibitors, diuretics, antiaggregants, sedatives, tranquillisers, anticonvulsants, anticoagulants, vitamins, agents for treating gastric and duodenal ulcers, proteolytic enzymes, healing factors and peptides.

3. A composition according to any of the previous Claims in which the silicone gum has a molecular weight greater than 200,000.

4. A composition according to Claim 3 wherein the silicone gum is a dimethylpolysiloxane encapped with dimethylhydroxysilyl units.

5. A composition according to any of the previous Claims in which the pharmaceutically active agent is present at a concentration of 0.1 to 10 wt. %.

6. A composition according to any of the previous Claims in which the silicone gum is present at a concentration of 0.5 to 2.85 wt. %.

7. A composition according to any of the previous Claims wherein the pharmaceutical excipient comprises a volatile excipient.

8. A composition according to Claim 7 wherein the pharmaceutical excipient is hexamethyldisiloxane.

9. A composition according to any of Claims 1-6 wherein the pharmaceutical excipient comprises those excipients used to form the composition into a delivery system selected from the group consisting of creams, lotions, ointments, emulsions and gels.

10. Use of a composition in accordance with any one of claims 1 to 9 as a pharmaceutical.

11. Use of a composition in accordance with any one of claims 1 to 9 in the manufacture of a medicament for a therapeutic application.

12. Use in accordance with claim 11 wherein the therapeutic application is selected from the group of an antibiotic, antiseptic, antifungal, antiacne, antiinflammatory, hormonal, anticancer, smoking cessation, cardiovascular, H₂ blocker, bronchodilator, analgesic, antiarrythmic, antihistamine, alpha blocker, beta blocker, ACE inhibitor, diuretic, antiaggregant, sedative, tranquilliser, anticonvulsant, anticoagulant, vitamin, gastric and duodenal ulcer, proteolytic enzyme, healing factor, cell growth nutrient or peptide application.

## Patentansprüche

1. Zusammensetzung zur topischen Zuführung eines pharmazeutisch aktiven Mittels in einen menschlichen oder tierischen Körper, enthaltend:
0,1 bis 25 Gew.-% pharmazeutisch aktives Mittel,
0,1 bis 3 Gew.-% Siliconharz und
72 bis 99,8 Gew.-% Arzneimittelträger.

2. Zusammensetzung nach Anspruch 1, worin das pharmazeutisch aktive Mittel ausgewählt ist aus der Gruppe bestehend aus diagnostischen Mitteln, Antibiotika, Antiseptika, fungiziden Mitteln, Antiaknemitteln. Hormonen, Krebsbekämpfungsmitteln, Mitteln zur Abgewöhnung des Rauchens, entzündungshemmenden Mitteln, Kreislaufmitteln, H₂-Blockern, Bronchienerweiterungsmitteln, schmerzlindernden Mitteln. Mitteln gegen Rhythmusstörungen, Alpha-1-Blockern, Beta-Blockern, ACE-Inhibitoren, harntreibenden Mitteln, Antiaggregationsmitteln, Sedativa, Beruhigungsmitteln, Krampfmitteln, Antikoagulantien, Vitaminen, Mitteln zur Behandlung von Magen- und Zwölffingerdarmgeschwüren, proteolytischen Enzymen, Heilfaktoren und Peptiden.

3. Zusammensetzung nach einem der vorstehenden Ansprüche, in welcher das Siliconharz ein Molekulargewicht von größer als 200.000 aufweist.

4. Zusammensetzung nach Anspruch 3, worin das Siliconharz ein Dimethylpolysiloxan ist, das mit Dimethylhydroxysilyleinheiten endverkappt ist.

5. Zusammensetzung nach einem der vorstehenden Ansprüche, in welcher das pharmazeutisch aktive Mittel in einer Konzentration von 0,1 bis 10 Gew.-% vorhanden ist.

6. Zusammensetzung nach einem der vorstehenden Ansprüche, in welcher das Siliconharz in einer Konzentration von 0,5 bis 2,85 Gew.-% vorhanden ist.

7. Zusammensetzung nach einem der vorstehenden Ansprüche, in welcher der Arzneimittelträger einen flüchtigen Träger enthält.

8. Zusammensetzung nach Anspruch 7, in welcher der Arzneimittelträger Hexamethyldisiloxan ist.

9. Zusammensetzung nach einem der Ansprüche 1-6, in welcher der Arzneimittelträger solche Träger umfasst, die verwendet werden, um aus der Zusammensetzung ein Zuführsystem zu bilden, ausgewählt aus der Gruppe bestehend aus Cremes, Lotionen, Salben, Emulsionen und Gels.

10. Verwendung einer Zusammensetzung nach einem der Ansprüche 1 bis 9 als Arzneimittel.

11. Verwendung einer Zusammensetzung nach einem der Ansprüche 1 bis 9 bei der Herstellung eines Medikaments für eine therapeutische Anwendung.

12. Verwendung nach Anspruch 11, worin die therapeutische Anwendung ausgewählt ist aus der Gruppe aus einer antibiotischen, antiseptischen, fungiziden, Antiakne-, entzündungshemmenden, hormonellen, Krebsbekämpfungs-, Rauchentwöhnungs-, Kreislauf-, H₂-Blocker-, Bronchienerweiterungs, schmerzlindernden, Antirhythmusstörungs-, Antihistamin-, Alpha-Blocker-. Beta-Blocker-, ACE-Inhibitor-, harntreibenden, Antiaggregations-, sedativen, Beruhigungsmittel-, Krampfmittel-, Antikoagulans-, Vitamin-, Magen- und Zwölffingerdarmgeschwürbehandlungs-, proteolytischen Enzym-, Heilfaktor-, Zellwachstumsnährstoff- oder Peptidanwendung.

## Revendications

1. Composition pour administrer un agent pharmaceutiquement actif en topique à un corps humain ou animal comprenant :
0,1 à 25 % en poids d'agent pharmaceutique actif ;
0,1 à 3 % en poids de gomme de silicone et
72 à 99,8% en poids d'excipient pharmaceutique.

2. Composition selon la revendication 1 dans laquelle l'agent pharmaceutique actif est choisi dans le groupe consistant en des agents de diagnostic, des antibiotiques, des antiseptiques, des antifongiques, des anti-acnéiques, des hormones, des anti-cancéreux, des compositions pour l'arrêt du tabagisme, des anti-inflammatoires, des agents cardio-vasculaires, des inhibiteurs H₂, des broncho-dilatateurs, des analgésiques, des anti-arythmiques, des alpha-1-bloqueurs, des bêta-bloqueurs, des inhibiteurs de l'ECA, des diurétiques, des anti-agrégants plaquettaires, des sédatifs, des tranquillisants, des anti-convulsivants, des anticoagulants, des vitamines, des agents pour traiter les ulcères gastriques et duodénaux, des enzymes protéolytiques, des facteurs de cicatrisation et des peptides.

3. Composition selon l'une quelconque des revendications précédentes dans laquelle la gomme de silicone a un poids moléculaire supérieur à 200 000.

4. Composition selon la revendication 3 dans laquelle la gomme de silicone est du diméthylpolysiloxane enrobé d'unités diméthylhydroxysilyle.

5. Composition selon l'une quelconque des revendications précédentes dans laquelle l'agent pharmaceutiquement actif est présent en une concentration de 0,1 à 10 % en poids.

6. Composition selon l'une quelconque des revendications précédentes dans laquelle la gomme de silicone est présente à une concentration de 0,5 à 2,85 % en poids.

7. Composition selon l'une quelconque des revendications précédentes dans laquelle l'excipient pharmaceutique comprend un excipient volatil.

8. Composition selon la revendication 7 dans laquelle l'excipient pharmaceutique est de l'hexaméthyldisiloxane

9. Composition selon l'une quelconque des revendications 1 à 6 dans laquelle l'excipient pharmaceutique comprend les excipients utilisés pour former la composition en un système d'administration choisi dans le groupe consistant en les crèmes, les lotions, les onguents, les émulsions et les gels.

10. Utilisation d'une composition selon l'une quelconque des revendications 1 à 9 comme produit pharmaceutique.

11. Utilisation d'une composition selon l'une quelconque des revendications 1 à 9 pour la fabrication d'un médicament pour une application thérapeutique.

12. Utilisation conformément à la revendication 11 dans laquelle l'application thérapeutique est choisie dans le groupe d'une application antibiotique, antiseptique, antifongique, anti-acnéique, anti-inflammatoire, hormonale, anti-cancéreuse, pour l'arrêt du tabagisme, cardio-vasculaire, d'inhibition H₂, broncho-dilatatrice, analgésique, anti-arythmique, antihistaminique, alpha-bloquante, bêta-bloquante, inhibitrice de l'ECA, diurétique, anti-agrégante, sédative, tranquillisante, anti-convulsivante, anticoagulante, de vitamine, contre l'ulcère gastrique et duodénal, d'enzyme protéolytique, de facteur de cicatrisation, de nutriment pour la croissance cellulaire ou de peptide.
